# EUROPEAN PATENT APPLICATION

(11) **EP 2 902 032 A1**
(43) Date of publication of application: **05.08.2015**
(21) Application number: 13842328.0
(22) Date of filing: 25.09.2013
(51) Int. Cl.: A61K 33/00, A61K 9/10, A61K 35/14, A61K 47/44, A61P 35/00, A61P 37/06

(54) **LIQUID MEDICINE HAVING CARBON DIOXIDE DISSOLVED THEREIN, AND THERAPEUTIC METHOD USING SAME**

(30) Priority: 26.09.2012 JP 2012212912
(71) Applicant: Neochemir, Inc., Kobe-shi Hyogo 651-0087 (JP); CO2BE Medical Engineering K.K., Hyogo 650-0047 (JP); National University Corporation Kobe University, Kobe-shi, Hyogo 657-8501 (JP)
(72) Inventor: TANAKA, Masaya, Kobe-shi Hyogo 651-0087 (JP); MIWA, Masahiko, Kobe-shi Hyogo 650-0047 (JP); FUJII, Masahiko, Kobe-shi Hyogo 657-8501 (JP); YAMAGUCHI, Masato, Kobe-shi Hyogo 657-8501 (JP)
(74) Representative: Sajda, Wolf E.
(86) International application number: PCT/JP2013/075951
(87) International publication number: WO 2014/050912

(57) **Abstract**

A liquid medicine of the present invention comprising liquid having carbon dioxide dissolved therein, the liquid being administered by using means of liquid injection, and a method of treatment of the present invention using a liquid medicine comprising liquid having carbon dioxide dissolved therein, the liquid being administered by using means of liquid injection can reduce or eliminate tumors with few side effects. When the liquid medicine of the present invention comprising liquid having carbon dioxide dissolved therein, the liquid being administered by using means of liquid injection, and the method of treatment of the present invention using a liquid medicine comprising liquid having carbon dioxide dissolved therein, the liquid being administered by using means of liquid injection are used in combination with surgical therapy, chemotherapy, radiotherapy, or immunotherapy of tumors, effects can be enhanced or side effects can be reduced compared with monotherapy or multidisciplinary therapy thereof.

## Description

### TECHNICAL FIELD

The present invention relates to a liquid medicine comprising liquid having carbon dioxide dissolved therein, said liquid being administered by using means of liquid injection, and a method of treatment using a liquid medicine comprising liquid having carbon dioxide dissolved therein, said liquid being administered by using means of liquid injection.

### BACKGROUND ART

Patent Document 1 describes that carbon dioxide is effective for treating symptoms etc. such as (1) to (10) as follows (See Patent Document 1):
(1) itching accompanying mucocutaneous diseases or mucocutaneous disorders such as athlete's foot, insect bite, atopic dermatitis, nummular eczema, xeroderma, seborrheic eczema, urticaria, prurigo, housewives' eczema, acne vulgaris, impetigo, folliculitis, carbuncle, furunculosis, phlegmon, pyoderma, psoriasis, ichthyosis, palmoplantar keratoderma, lichen, pityriasis, wound, burn, rhagades, erosion and chilblain;
   mucocutaneous injuries such as decubitus ulcer, wound, burn, angular stomatitis, stomatitis, skin ulcer, rhagades, erosion, chilblain and gangrene;
(2) incomplete takes of skin graft, skin flap, etc.;
(3) dental diseases such as gingivitis, alveolar pyorrhea, denture ulcer, nigricans gingiva, and stomatitis;
(4) skin ulcers, cryesthesia and numbness caused by peripheral circulatory disorders such as thromboangitis obliterans, arteriolosclerosis obliterans, diabetic peripheral circulatory disorder, and varicosis in lower extremity;
(5) musculoskeletal diseases such as chronic rheumatoid arthritis, cervico-omo-brachial syndrome, myalgia, arthralgia and lumbago;
(6) nervous system diseases such as neuralgia, polyneuritis and subacute myelo-optic neuropathy;
(7) keratoses such as psoriasis, corns, callouses, ichthyosis, palmoplantar keratoderma, lichen, and pityriasis;
(8) suppurative skin diseases such as acne vulgaris, impetigo, folliculitis, carbuncles, furuncules, phlegmon, pyoderma and suppurative eczema;
(9) suppression of hair regrowth after depilation (treatment of unwanted hair); and
(10) cosmetic troubles with the skin or hair such as freckles, rough skin, muddy complexion, faded skin complexion and loss of hair gloss, etc.

In order to achieve the amelioration of the above mentioned symptoms, as means for absorbing carbon dioxide, a composition for preparing a carbon dioxide preparation for external use (see Patent Documents 2 and 3), a carbon dioxide composition for external use (see Patent Document 4), a composition for preparing a carbon dioxide gel for external use (see Patent Document 5), a material for formation of a carbon dioxide external preparation (see Patent Document 6), and a carbon dioxide external administration device (see Patent Documents 7 and 8) are disclosed.

Also use of means for absorbing carbon dioxide to strengthen muscle is disclosed (Patent Document 9).

Carbon dioxide in a gaseous state as it is, or dispersed as fine bubbles in a physiological saline solution is injected into blood vessels to serve as a negative contrast medium. Even though the negative contrast media have been used for patients with various diseases including cancers and autoimmune diseases, the effectiveness for these diseases haven't been reported yet.

However, a liquid medicine comprising liquid having carbon dioxide dissolved therein, said liquid being administered by using means of liquid injection, and use, as a medicine, of liquid having carbon dioxide dissolved therein, said liquid being administered by means of liquid injection haven't been known.

In the present invention, a liquid medicine comprising liquid having carbon dioxide dissolved therein, said liquid being administered by using means of liquid injection is used for both prevention and treatment of diseases including at least cancers and autoimmune diseases.

In the present invention, the objective of a method of treatment using liquid having carbon dioxide dissolved therein, said liquid being administered by using means of liquid injection, includes tumors and autoimmune diseases.

In general there are three major methods of treating tumors, which are surgical therapy, chemotherapy and radiation therapy. Also the modern policy of treating tumors is multidisciplinary therapy, in which multiple methods for treating tumors are combined. Further, immunotherapy is now attracting attention as the fourth method for treating tumors.

Surgical therapy has developed since the old days as a remedy for treating tumors and is a core of the multidisciplinary therapy at present. On the contrary, rapid proliferation of tumor cells and/or metastasis are sometimes observed after surgical operation, and maintaining the quality of life (QOL) is often hindered by loss of vital function accompanied with resection of the tumor or dysfunction after surgery.

Also physical strength for the surgery is required for a patient receiving this therapy, and it is difficult to apply the therapy to an aged person or a patient who has decreased physical strength through a long treatment.

In chemotherapy, a chemical substance (an anticancer agent) is administrated to suppress tumor cell division and kill the tumor cell. After injection or oral administration, the anticancer agent is delivered from head to feet through blood vessels, and attacks tumor cells luring in the body. It is possible to attack antitumor cells throughout the body and therefore effective in systemic treatment.

However, an anticancer agent not only attacks tumor cells but has side effects to damage normal cells, and there is no selective toxicity not to affect normal cells in the anticancer agent available at present. In order to improve the selective toxicity, a lot of studies are now in progress but no anticancer agent without side effects or no means to prevent side effects completely is known.

In radiotherapy, tumor cell division is inhibited and proliferation of tumor cells is suppressed by irradiation with radiations such as X rays or gamma rays. But the radiation gives damage to not only tumor cells but also normal cells. What happens is that methods of irradiation are continuously studied so that they give the maximal effect only to the tumor cells. Indeed, the radiotherapy is not free from side effects and some side effects are observed just after treatment

(acute stage), and other side effects come out in a half year to several years (late stage). In general, typical examples of the side effects include inflammation of the skin or mucosa, bone-marrow disorder, fatigue, and anorexia. More or less, symptoms which are thought to be side effects are observed in most cases.

In immunotherapy, immune cells or proteins attack tumor cells by activating the patient's immune system so that tumors are treated. Usually lentinan, Krestin (trade name), Picibanil (trade name), sizofiran, BCG or tumor vaccine etc. are used to activate the immune system. Although side effects are not strong, therapeutic effects are insufficient. Also a therapeutic method in which the patient's lymphocytes are taken out of the body, cultivated, activated, and put back to the inside of the body again to kill tumor cells, and a therapeutic method in which tumor cells are directly attacked by anti-tumor antibodies are studied. However, their effects are not reliable, and expensive treatment cost is required.

An autoimmune disease is a disease in which the body's protective mechanism, which ought to eliminate a foreign material, attacks self-tissues. For treatment, a non-steroidal anti-inflammatory agent, a steroidal anti-inflammatory agent, an immunosuppressant and the like are used. Alternatively, plasma exchange therapy is carried out. Permanent cure is difficult in all of them, and side effects have not been overcome.

### PRIOR ART REFERENCES

### PATENT DOCUMENTS

Patent Document 1: JP 2000-319 187 A
Patent Document 2 WO 2002/80941 A1
Patent Document 3 WO 2006/80398 A1
Patent Document 4 WO 2003/57228 A1
Patent Document 5 WO 2005/16290 A1
Patent Document 6 WO 2004/4745 A1
Patent Document 7 WO 2004/2393 A1
Patent Document 8 WO 2007/112726 A1
Patent Document 9 JP 2009-120 606 A.

### SUMMARY OF THE INVENTION

### PROBLEM TO BE SOLVED BY THE INVENTION

Objects of the present invention are to provide a liquid medicine comprising liquid having carbon dioxide dissolved therein, said liquid being administered by using means of liquid injection, and a method of treatment using a liquid medicine comprising liquid having carbon dioxide dissolved therein, said liquid being administered by using means of liquid injection. Their effects are achieved simply and safely.

### MEANS FOR SOLVING THE PROBLEM

Inventors of the present invention have found that a tumor is reduced or disappears, and tumor metastasis is suppressed by administering liquid having carbon dioxide dissolved therein, said liquid being administered by using means of liquid injection, and completed the present invention.

That is, the present invention is a liquid medicine comprising liquid having carbon dioxide dissolved therein, said liquid being administered by using means of liquid injection, by which (1) a tumor is reduced or disappears, (2) tumor metastasis is suppressed, and (3) an autoimmune disease is ameliorated.

Also, the present invention is a method of treatment using a liquid medicine comprising liquid having carbon dioxide dissolved therein, said liquid being administered by using means of liquid injection, by which (1) a tumor is reduced or disappears, (2) tumor metastasis is suppressed, and (3) an autoimmune disease is ameliorated.

By the way, carbon dioxide is considered to exhibit its actions by the Bohr effect that carbon dioxide enters red blood cells, and liberates oxygen adsorbed onto hemoglobin, it is carbon dioxide dissolved in blood and the like that can penetrate cell membrane. Gaseous carbon dioxide cannot pass through the cell membrane which is filled with liquid unless it is dissolved in blood or the like. Moreover, the amount by mole of carbon dioxide contained in a saturated aqueous solution of carbon dioxide is about 100 times compared to that of gaseous carbon dioxide with the same volume. Therefore, it is thought that conventionally used negative contrast media such as carbon dioxide is thought not to show the effects as in the present invention.

In the present invention, both of a malignant tumor and a benign tumor are included in the tumor.

Examples of the malignant tumor include malignant tumors of brain nerves such as glioma and meningioma; malignant tumors of oral cavity, nose, nasal cavity, larynx or pharynx such as tongue cancer, gingival cancer, malignant lymphoma, malignant melanoma, maxillary cancer, nose cancer, nasal cavity cancer, laryngeal cancer and pharyngeal cancer; malignant tumors of thyroid such as thyroid papillary carcinoma, follicular thyroid cancer and medullary thyroid carcinoma; malignant tumors of respiratory tract such as squamous cell carcinoma, adenocarcinoma, alveolar cell carcinoma, large cell anaplastic carcinoma, small cell anaplastic carcinoma and carcinoid; malignant tumors of breast such as breast cancer, mammary Paget's disease and breast sarcoma; malignant tumors of blood such as acute myeloid leukemia, acute promyelocytic leukemia, acute myelomonocytic leukemia, acute monocytic leukemia, acute lymphocytic leukemia, acute undifferentiated leukemia, chronic myeloid leukemia, chronic lymphocytic leukemia, adult T-cell leukemia, malignant lymphoma, multiple myeloma and primary macroglobulinemia; malignant tumors of digestive organ such as esophageal cancer, stomach cancer, stomach-large intestine leiomyosarcoma, stomach-intestine malignant lymphoma, pancreatic-gallbladder cancer, duodenal cancer, colon cancer, primary liver cancer and hepatoblastoma; malignant tumors of female genital such as uterine epithelium cancer, uterine cervix squamous epithelium cancer, uterine adenocarcinoma, uterine gland squamous epithelium cancer, uterine body adenoacanthoma, uterine sarcoma, uterine cancer sarcoma, destructive hydatidiform mole, malignant chorioepithelioma of uterus, malignant melanoma of uterus, ovarian cancer and mesodermal mixed tumor; malignant tumors of digestive organ such as kidney cancer, transitional cell cancer of renal pelvis, ureter transitional cell carcinoma, bladder papillary carcinoma, bladder transitional cell carcinoma, prostate cancer, urinary tract squamous cell carcinoma, urethral adenocarcinoma and Wilms' tumor; malignant tumors of musculoskeletal such as rhabdomyosarcoma, fibrosarcoma, osteosarcoma, chondrosarcoma, synovial sarcoma, mucus sarcoma, liposarcoma, Ewing sarcoma and multiple myeloma; malignant tumors of skin such as skin squamous cell carcinoma, basal cell skin cancer, skin Bowen's disease, Paget's disease of skin and cutaneous malignant melanoma; and malignant tumors of body cavity such as malignant mesothelioma cancer, malignant melanoma, metastatic adenocarcinoma, metastatic squamous cell carcinoma, metastatic sarcoma, leukemia, malignant lymphoma and neuroblastoma; or benign tumors of brain such as meningioma, pituitary adenoma and nerve sheath tumor; benign tumors of dermal and subcutaneous tissue such as nevus, suspended fibroma, hemangioma, vascular birthmark, lymphangioma, pyogenic granuloma, seborrheic keratosis, dermatofibroma, keratoacanthoma, keloid, lipoma, brown lipoma, neurofibromatosis, schwannoma and atheroma; benign tumors of bone such as osteocartilaginous exostosis, chondroma, chondroblastoma, osteoid osteoma and giant cell tumor; benign tumors of intestinal such as lipoma, fibroids and polyp; benign tumors of liver such as hepatocellular adenoma and bile duct adenoma; benign tumors of bile duct such as papilloma and villous adenoma; benign tumors of ear such as ear mushroom and cholesteatoma; and benign tumors of oral cavity or salivary gland such as benign pleomorphic adenoma, monomorphic adenoma, oncocytoma, papillary cystadenoma lymphoma and ameloblastoma.

In the present invention, an autoimmune disease is a disorder in which the immune system recognizes the body's own tissues and cells as foreign substances, attacks and damages them. Organ-specific autoimmune diseases and organ non-specific autoimmune diseases are included therein.

Examples of the organ-specific autoimmune diseases include pernicious anemia, Addison's disease, insulin-dependent diabetes mellitus, ulcerative colitis, rapidly progressive gromerulonephritis, megaloblastic anemia, good pasture syndrome, primary hypothyroidism, primary biliary cirrhosis, primary sclerosing cholangitis, primary myxedema, thyrotoxicosis, acquired epidermolysis bullosa, relapsing polychondritis, autoimmune atrophic gastritis, autoimmune optic neuropathy, autoimmune pancreatitis, autoimmune hemolytic anemia, autoimmune neutropenia, pemphigus vargalis, juvenile diabetes, myasthenia gravis, lens-induced uveitis, bullous pemphigoid, climacterium precox, aortitis syndrome, multiple screlosis, male infertility, idiopathic thrombocytopenic purpura, Basedow's disease, paroxysmal hemoglobinuria, Hashimoto's disease, Harada's disease, chronic active hepatitis, pemphigoid, and the like.

Examples of the organ non-specific autoimmune diseases include discoid lupus erythematosus, systemic lupus erythematosus, scleroderma, Crohn's disease, antiphospholipid antibody syndrome, mixed connective-tissue disease, sarcoidosis, Sjogren's syndrome, polymyositis, polyangiitis, Bechet's disease, chronic rheumatoid arthritis, and the like.

### EFFECT OF THE INVENTION

The liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection are useful because they can reduce or eliminate tumors, suppress metastasis of tumors, and ameliorate autoimmune diseases with few side effects.

When the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection are used in combination with one or two or more selected from surgical therapy, chemotherapy, radiotherapy, and immunotherapy of tumors, anti-tumor effects can be enhanced or side effects can be reduced compared with a monotherapy or multidisciplinary therapy thereof.

More specifically, in the surgical therapy of tumors, the combined use of the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection suppresses rapid growth or metastasis of tumor cells, or reduces tumor cells compared to the surgical therapy alone, thus making it possible to improve the treatment results.

In the chemotherapy of tumors, the combined use of the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection gives the same effects with a smaller dose of a chemotherapeutic agent compared to administration of the chemotherapeutic agent alone, so that side effects are reduced. If the dose of the chemotherapeutic agent is the same as that of the chemotherapeutic agent alone, better effects are obtained.

In the radiotherapy of tumors, the combined use of the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection gives the same effects with a smaller dose of radiation compared to radiation alone, so that side effects are reduced. If the dose of radiation is the same as that of radiation alone, better effects are obtained.

In the immunotherapy of tumors, the combined use of the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection gives the same effects with a smaller dose of an immunotherapeutic agent (including a living body portion such as activated lymphocytes) and the like compared to the immunotherapy alone, so that side effects are reduced. If the dose of the immunotherapeutic agent and the like is the same as that of immunotherapy alone, better effects are obtained.

The liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection can easily treat tumors even in medical institutions with no facilities for hospitalization.

With the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection, since carbon dioxide as an active ingredient is inexpensive, the cost required for the treatment is less than any of the surgical therapy, chemotherapy, radiotherapy, and immunotherapy of tumors.

The liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection not only can improve symptoms of autoimmune diseases, but also can prevent and cure the diseases themselves. More specifically, for example, abnormal growth of connective tissues due to chronic inflammation and the like are suppressed or normalized, so that fibrosis of organs and tissues is ameliorated. Therefore, functional recovery and maintenance of the organs and tissues are enabled.

When the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection are used in combination with one or two or more of a non-steroidal anti-inflammatory agent, a steroidal anti-inflammatory agent, an immunosuppressant, and a plasma exchange therapy, the degree of improvement in symptoms of autoimmune diseases becomes better, or side effects can be reduced compared to their single or combined use.

The liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection can also be applied to animals.

### BRIEF DESCRIPTION OF THE DRAWINGS

- FIG. 1: is a drawing that shows antitumor effects on the implanted tumor in rabbits by a liquid medicine comprising liquid having carbon dioxide dissolved therein, said liquid being administered by using means of liquid injection;
- FIG. 2: is a drawing that shows antitumor effects on the implanted tumor in rabbits by a liquid medicine comprising liquid having carbon dioxide dissolved therein, said liquid being administered by using means of liquid injection;
- FIG. 3: is a drawing that shows safety to tumor-implanted rabbits (body weight changes) of a liquid medicine comprising liquid having carbon dioxide dissolved therein, said liquid being administered by using means of liquid injection rabbits in the implanted tumor;
- FIG. 4: is a drawing that shows antitumor effects on the implanted tumor cells in rabbits by a liquid medicine comprising liquid having carbon dioxide dissolved therein, said liquid being administered by using means of liquid injection;
- FIG. 5: is a drawing that shows safety to tumor-implanted rabbits (body weight changes) of a liquid medicine comprising liquid having carbon dioxide dissolved therein, said liquid being administered by using means of liquid injection;
- FIG. 6: is a drawing that shows safety (body temperature changes) to a healthy pig of a liquid medicine comprising liquid having carbon dioxide dissolved therein, said liquid being administered by using means of liquid injection;
- FIG. 7: is a drawing that shows safety to a healthy pig (arterial blood pH changes) of a liquid medicine comprising liquid having carbon dioxide dissolved therein, said liquid being administered by using means of liquid injection;
- FIG. 8: is a drawing that shows safety to a healthy pig (changes in partial pressure of carbon dioxide in arterial blood) of a liquid medicine comprising liquid having carbon dioxide dissolved therein, said liquid being administered by using means of liquid injection; and
- FIG. 9: is a drawing that shows safety to a healthy pig (changes in partial pressure of oxygen in arterial blood) of a liquid medicine comprising liquid having carbon dioxide dissolved therein, said liquid being administered by using means of liquid injection.

### EMBODIMENTS OF THE INVENTION

In the liquid medicine of the present invention comprising liquid having carbon dioxide dissolved therein, said liquid being administered by using means of liquid injection, the liquid that dissolves carbon dioxide preferably has a carbon dioxide solubility of about 1 mg/L or more at 25 °C, a viscosity in which about 0.1 mL or more of the liquid is forced out of an injection needle or a catheter tube for one minute, and an osmotic pressure of about 280 milliosmol which is the same as an isotonic solution.

When the liquid in which carbon dioxide is dissolved contains water, the liquid medicine of the present invention is preferably neutral or acidic.

Carbon dioxide is dissolved in water, oil, alcohol, and amine. Any of these may be used as long as it is a liquid which has high safety and in which the solubility of carbon dioxide, viscosity and osmotic pressure satisfy the above conditions. As the liquid that dissolves carbon dioxide, physiological saline solution is preferred. Further, the liquid may contain a substance such as hemoglobin which reversibly binds carbon dioxide. Therefore, as the liquid that dissolves carbon dioxide, blood compatible with the patient's blood type is also preferred. Of course, the patient's own blood may be used. The blood compatible with the patient's blood type means blood which does not cause any unfavorable reactions such as rejection.

Although oil may be used as the liquid that dissolves carbon dioxide, if the liquid is entirely composed of oil, side effects such as a blood clot could be caused when the liquid is injected into blood vessels. Therefore, the liquid is preferably used in combination with water. In that case, a surfactant is used so that the liquid serves as emulsion. Fat emulsion is preferable as emulsion. Commercially available fat emulsion for intravenous injection containing 10 % or 20 % purified soybean oil as fat emulsion may be used, but the present invention is not limited thereto.

Methods for dissolving carbon dioxide in the liquid are not particularly limited. Carbon dioxide may be blown into the liquid through a tube and the like. Alternatively, a reaction of biocompatible acid and carbonate in a liquid to generate carbon dioxide may be used.

Carbon dioxide concentration in the liquid is preferably 60 ppm or more, and more preferably 100 ppm or more. The higher the concentration of carbon dioxide, the better, and there is no upper limit. The liquid medicine of the present invention comprising liquid having carbon dioxide dissolved therein, said liquid being administered by using means of liquid injection may contain a bubble(s)_of carbon dioxide if the liquid has a given carbon dioxide concentration or higher.

It is preferable that carbon dioxide is less contaminated with a substance causing side effects and the like, and highly-pure liquefied carbon dioxide is more preferable.

The injection means of the liquid medicine of the present invention comprising liquid having carbon dioxide dissolved therein, said liquid being administered by using means of liquid injection is not particularly limited as long as it is means that can inject a given amount of the liquid into a living body. An active injection method such as a syringe or a pump may be used.

With regard to the injection amount of the liquid medicine of the present invention comprising liquid having carbon dioxide dissolved therein, said liquid being administered by using means of liquid injection, the more, the more preferable. The injection amount is to be not more than the maximum amount the patient can tolerate in accordance with the patient's age, body weight, respiratory function, kidney function and the like.

The liquid medicine of the present invention comprising liquid having carbon dioxide dissolved therein, said liquid being administered by using means of liquid injection may be injected into an affected area directly, or a tissue in the vicinity of the affected area etc. It is preferably injected into a feeding vessel of the affected area. The blood flow of the feeding vessel may be inhibited or reduced so that the liquid medicine of the present invention may stay in the vicinity of the affected area for as long hours as possible.

In case of bladder tumor, using an urethral catheter and the like, the liquid medicine of the present invention comprising liquid having carbon dioxide dissolved therein, said liquid being administered by using means of liquid injection may be injected into the bladder. Circulating a liquid medicine in which carbon dioxide is dissolved at a high concentration within the bladder by using a liquid circulation device provided with a liquid medicine injection tube, a liquid medicine recovery tube, and a device for dissolving carbon dioxide in liquid enables an effective tumor therapy.

When the liquid medicine of the present invention comprising liquid having carbon dioxide dissolved therein, said liquid being administered by using means of liquid injection is used in combination with surgical therapy, chemotherapy, radiotherapy, immunotherapy of tumors, or a therapy of autoimmune diseases, said liquid medicine may be injected each time each therapy (including a multidisciplinary therapy of tumors) is performed, or alternatively said liquid medicine may be injected on the day when each therapy (including multidisciplinary therapy of tumors) is not performed. When used in combination with the chemotherapy or immunotherapy of tumors, or the therapy of autoimmune diseases, a chemotherapeutic agent or an immunotherapeutic agent, or a therapeutic agent for autoimmune diseases may be concurrently contained in the liquid medicine of the present invention.

The surgical therapies used in combination with the liquid medicine of the present invention comprising liquid having carbon dioxide dissolved therein, said liquid being administered by using means of liquid injection include, for example, extended radical operation, reduction surgery, functional preservation operation, endoscopic surgery, celoscope surgery and the like.

The radiation therapies used in combination with the liquid medicine of the present invention comprising liquid having carbon dioxide dissolved therein, said liquid being administered by using means of liquid injection include gamma-knife therapy wherein a subject is irradiated with gamma rays, cyberknife therapy wherein a subject is irradiated with X-rays, and the like. Both of these are stereotactic radiotherapies using Linac (a linear accelerator).

Chemotherapeutic agents used in combination with the liquid medicine of the present invention comprising liquid having carbon dioxide dissolved therein, said liquid being administered by using means of liquid injection include anticancer agents such as molecular target drugs, alkylating agents, antimetabolites, plant alkaloids, antitumor antibiotics, platinum-containing drugs, hormonal agents, and the like.

The molecular target drugs used in combination as the chemotherapeutic agent include ibritumomab tiuxetan, imatinib, erlotinib, gefitinib, gemtuzumab ozogamicin, sunitinib, cetuximab, sorafenib, dasatinib, tamibarotene, trastuzumab, tretinoin, panitumumab, bevacizumab, bortezomib and rituximab.

The alkylating agents used in said combined chemotherapy include ifosfamide, cyclophosphamide, dacarbazine, temozolomide, nimustine, busulfan and melphalan.

The antimetabolites used in combination as the chemotherapeutic agent include enocitabine, capecitabine, carmofur, gemcitabine, cytarabine, tegafur, tegafur-uracil, nelarabine, fluorouracil, fludarabine, pemetrexed, pentostatin and methotrexate.

The plant alkaloids used in combination as the chemotherapeutic agent include irinotecan, etoposide, sobuzoxane, docetaxel, nogitekan, paclitaxel, vinorelbine, vincristine, vindesine and vinblastine.

The anticancer antibiotics used in combination as the chemotherapeutic agent include actinomycin D, aclarubicin, idarubicin, epirubicin, daunorubicin, doxorubicin (adriamycin), pirarubicin, bleomycin, peplomycin, mitomycin C and mitoxantrone.

The platinum-containing drugs used in combination as the chemotherapeutic agent include oxaliplatin, carboplatin, cisplatin and nedaplatin.

The hormonal agents used in combination as the chemotherapeutic agent include anastrozole, exemestane, ethinyl estradiol, chlormadinone, goserelin, tamoxifen, bicalutamide, flutamide, buredonizoron, leuprorelin and letrozole.

In the combined use of the chemotherapeutic agent, a hydrophilic chemotherapeutic agent, or a hydrophobic chemotherapeutic agent may be used by being dissolved or suspended together with carbon dioxide in the liquid medicine of the present invention, comprising liquid having carbon dioxide dissolved therein, said liquid being administered by using means of liquid injection in accordance with the physical properties of said liquid.

In that case, the dose of the chemotherapeutic agent is reduced than the standard dose, and, while complementing the anti-tumor effect with carbon dioxide, side effects can be reduced. Alternatively, when the dose of the chemotherapeutic agent is the same as the standard one, although usual side effects are observed, the anti-tumor effect can also be more enhanced with carbon dioxide.

The immunotherapies used in combination with the liquid medicine of the present invention comprising liquid having carbon dioxide dissolved therein, said liquid being administered by using means of liquid injection include cytokine therapy, immunomodulating therapy, and the like as active immunotherapies as well as advanced NK cell therapy and activated lymphocyte therapy as passive immunotherapies (immune cell therapies).

The cytokine therapies used in said combined immunotherapy include administration of interleukins such as interleukin 2, and interleukin 12, interferons such as interferon-α, interferon-β, interferon-γ, tumor necrosis factors such as TNF-α, and the like.

The immunomodulating therapies used in said combined immunotherapy include administration of immunomodulating agents such as BCG, tuberculosis bacteria, ubenimex, Picibanil, lentinan, and Krestin.

In the combined use of the immunotherapy, a hydrophilic immunotherapeutic agent, or a hydrophobic immunotherapeutic agent may be used by being dissolved or suspended together with carbon dioxide in a liquid medicine of the present invention comprising liquid having carbon dioxide dissolved therein, said liquid being administered by using means of liquid injection in accordance with the physical properties of said liquid.

In that case, the dose of the immunotherapeutic agent is reduced than the standard dose, and, while complementing the anti-tumor effect with carbon dioxide, side effects can be reduced. Alternatively, when the dose of the immunotherapeutic agent is the same as the standard one, although usual side effects are observed, the anti-tumor effect can also be more enhanced with carbon dioxide.

Examples of the non-steroidal anti-inflammatory agents used in combination with the liquid medicine of the present invention comprising liquid having carbon dioxide dissolved therein, said liquid being administered by using means of liquid injection include salicylic acid-based anti-inflammatory agents such as acetylsalicylic acid, salicylamide, sodium salicylate, diflunisal, and ethenzamide; arylacetic acid-based anti-inflammatory agents such as acemetasin, anfenac sodium, indometacin, etodolac, diclofenac sodium, sulindac, nabumetone, fenbufen, ploglumetacin maleate, and mofezolac; oxicam-based anti-inflammatory agents such as ampiroxicam, tenoxicam, piroxicam, meloxicam, and lornoxicam; propionic acid-based anti-inflammatory agents such as alminoprofen, ibuprofen, oxaprozin, ketoprofen, zaltoprofen, tiaprofenic acid, naproxen, fenoprofen, flurbiprofen, pranoprofen, and loxoprofen sodium; fenamic acid-based anti-inflammatory agents such as tolfenamic acid, flufenamate aluminum, and mefenamic acid; pyrimidine-based anti-inflammatory agents such as bucolome; coxib-based anti-inflammatory agents such as celecoxib, valdecoxib, lumiracoxib, and parecoxib sodium; and basic anti-inflammatory agents such as emorfazone, epirizole, and tiaramide hydrochloride.

Examples of the steroidal anti-inflammatory agents include hydrocortisone succinate, hydrocortisone sodium succinate, prednisolone sodium succinate, methylprednisolone succinate, methylprednisolone sodium succinate, dexamethasone, triamcinolone, triamcinolone acetonide, dexamethasone palmitate, hydrocortisone, prednisolone, betamethasone, methylprednisolone, dexamethasone sodium phosphate, hydrocortisone sodium phosphate, prednisolone sodium phosphate, betamethasone sodium phosphate, cortisone acetate, dexamethasone acetate, paramethasone acetate, halopredone acetate, fludrocortisone acetate, methylpredonisolone acetate, and the like.

Examples of the immunosuppressants include the above steroidal anti-inflammatory agents, alkylating agents such as cyclophosphamide, cytotoxic antibiotics such as anthracycline, dactinomycin, mitomicin c, mithramycin, and bleomycin; cytokine inhibitors such as adalimumab, infliximab, etanercept, and tocilizumab; metabolic antagonists such as azathioprine, mycophenolic acid, mycophenolate mofetil, leflunomide, mercaptopurine, and methotrexate; specific lymphocyte signal transduction inhibitors such as evelolimus, cyclosporine, sirolimus, tacrolimus, and temsirolimus; antibodies against IL-2 receptor protein such as CD3 and CD25; and cytokines such as interferon.

In the combined use of the non-steroidal anti-inflammatory agent, the steroidal anti-inflammatory agent, or the immunosuppressant (hereinafter referred to as agent for autoimmune diseases), a hydrophilic agent for autoimmune diseases, or a hydrophobic agent for autoimmune diseases may be used by being dissolved or suspended together with carbon dioxide in the liquid medicine of the present invention comprising liquid having carbon dioxide dissolved therein, said liquid being administered by using means of liquid injection in accordance with the physical properties of said liquid.

In that case, the dose of the agent for autoimmune diseases is reduced than the standard dose, and, while complementing or enhancing the effect with carbon dioxide, side effects can be reduced. Alternatively, when the dose of the agent for autoimmune diseases is the same as the standard one, the effect can also be more enhanced with carbon dioxide.

In the method of treatment of the present invention using a liquid medicine comprising liquid having carbon dioxide dissolved therein, said liquid being administered by using means of liquid injection, the liquid that dissolves carbon dioxide preferably has a carbon dioxide solubility of about 1 mg/L or more at 25 °C, a viscosity in which about 0.1 mL or more of the liquid is forced out of an injection needle or a catheter tube for one minute, and an osmotic pressure of about 280 milliosmol which is the same as an isotonic solution.

When the liquid in which carbon dioxide is dissolved contains water, the liquid medicine of the present invention is preferably neutral or acidic.

Carbon dioxide dissolves in water, oil, alcohol, and amine. Any of these may be used as long as it has high safety and in which the solubility of carbon dioxide, viscosity and osmotic pressure satisfy the above conditions. As the liquid that dissolves carbon dioxide, a physiological saline solution is preferred. Further, the liquid may contain a substance such as hemoglobin which reversibly binds carbon dioxide.

Therefore, as the liquid that dissolves carbon dioxide, blood compatible with the patient's blood type is also preferred. Of course, the patient's own blood may be used. The blood compatible with the patient's blood type means blood which does not cause any unfavorable reactions such as rejection.

Although oil may be used as the liquid that dissolves carbon dioxide, if the liquid is entirely composed of oil, side effects such as a blood clot could be caused when the liquid is injected into blood vessels. Therefore, the liquid is preferably used in combination with water. In that case, a surfactant is used so that the liquid serves as emulsion. Fat emulsion is preferable as emulsion. Commercially available fat emulsion for intravenous injection containing 10 % or 20 % purified soybean oil as fat emulsion may be used, but the present invention is not limited thereto.

Methods for dissolving carbon dioxide in the liquid are not particularly limited. Carbon dioxide may be blown into the liquid through a tube and the like. Alternatively, a reaction of biocompatible acid and carbonate in a liquid to generate carbon dioxide may be used.

Carbon dioxide concentration in the liquid is preferably 60 ppm or more, and more preferably 100 ppm or more. The higher the concentration of carbon dioxide, the better, and there is no upper limit. In the method of treatment of the present invention using a liquid medicine comprising liquid having carbon dioxide dissolved therein, said liquid being administered by using means of liquid injection, said liquid may contain a bubble(s) of carbon dioxide if the liquid has a given carbon dioxide concentration or higher.

It is preferable that carbon dioxide is less contaminated with a substance causing side effects and the like, and highly pure liquefied carbon dioxide is more preferable.

The injection means in the method of treatment of the present invention using a liquid medicine comprising liquid having carbon dioxide dissolved therein, said liquid being administered by using means of liquid injection is not particularly limited as long as it is means that can inject a given amount of the liquid into a living body. An active injection method such as a syringe or a pump may be used.

With regard to the injection amount in the method of treatment of the present invention using a liquid medicine comprising liquid having carbon dioxide dissolved therein, said liquid being administered by using means of liquid injection, the more, the more preferable. The injection amount is to be not more than the maximum amount the patient can tolerate in accordance with the patient's age, body weight, respiratory function, kidney function and the like.

In the method of treatment using a liquid medicine of the present invention comprising liquid having carbon dioxide dissolved therein, said liquid being administered by using means of liquid injection, the liquid medicine may be injected into an affected area directly, or a tissue in the vicinity of the affected area. It is preferably injected into a feeding vessel of the affected area. The blood flow of the feeding vessel may be inhibited or reduced so that the liquid medicine of the present invention may stay in the vicinity of the affected area for as long hours as possible.

In case of bladder tumor, using an urethral catheter and the like, liquid having carbon dioxide dissolved therein may be injected. Circulating a liquid medicine in which carbon dioxide is dissolved at a high concentration within the bladder by using a liquid circulation device provided with a liquid medicine injection tube, a liquid medicine recovery tube, and a device for dissolving carbon dioxide in a liquid enables an effective tumor therapy.

When the method of treatment of the present invention using a liquid medicine comprising liquid having carbon dioxide dissolved therein, said liquid being administered by using means of liquid injection is used in combination with surgical therapy, chemotherapy, radiotherapy, or immunotherapy of tumors, said liquid medicine may be used each time each therapy (including multidisciplinary therapy) is performed, or alternatively, said liquid medicine may be used on the day when they (including multidisciplinary therapy) are not performed. When used in combination with chemotherapy or immunotherapy of tumors, a chemotherapeutic agent, or an immunotherapeutic agent may be concurrently contained in the liquid containing carbon dioxide.

The surgical therapies used in combination with the method of treatment of the present invention using a liquid medicine comprising liquid having carbon dioxide dissolved therein, said liquid being administered by using means of liquid injection include, for example, extended radical operation, reduction operation, function preserving operation, endoscopic surgery, celoscope surgery, and the like.

The radiation therapies used in combination with the method of treatment of the present invention using a liquid medicine comprising liquid having carbon dioxide dissolved therein, said liquid being administered by using means of liquid injection include, for example, gamma-knife therapy wherein a subject is irradiated with gamma rays, cyberknife therapy wherein a subject is irradiated with X-rays, and the like. Both of these are stereotactic radiotherapies using Linac (a linear accelerator).

The chemotherapies used in combination with the method of treatment of the present invention using a liquid medicine comprising liquid having carbon dioxide dissolved therein, said liquid being administered by using means of liquid injection include administration of anticancer agents such as molecular target drugs, alkylating agents, antimetabolites, plant alkaloids, antitumor antibiotics, platinum-containing drugs, hormonal agents, and biological response modifiers.

The molecular target drugs used in said combined chemotherapy include ibritumomab tiuxetan, imatinib, erlotinib, gefitinib, gemtuzumab ozogamicin, sunitinib, cetuximab, sorafenib, dasatinib, tamibarotene, trastuzumab, tretinoin, panitumumab, bevacizumab, bortezomib and rituximab.

The alkylating agents used in said combined chemotherapy include ifosfamide, cyclophosphamide, dacarbazine, temozolomide, nimustine, busulfan and melphalan.

The antimetabolites used in said combined chemotherapy include enocitabine, capecitabine, carmofur, gemcitabine, cytarabine, tegafur, tegafur-uracil, nelarabine, fluorouracil, fludarabine, pemetrexed, pentostatin and methotrexate.

The plant alkaloids used in said combined chemotherapy include irinotecan, etoposide, sobuzoxane, docetaxel, nogitekan, paclitaxel, vinorelbine, vincristine, vindesine and vinblastine.

The anticancer antibiotics used in said combined chemotherapy include actinomycin D, aclarubicin, idarubicin, epirubicin, daunorubicin, doxorubicin (adriamycin), pirarubicin, bleomycin, peplomycin, mitomycin C and mitoxantrone.

The platinum-containing drugs used in said combined chemotheray include oxaliplatin, carboplatin, cisplatin and nedaplatin.

The hormonal agents used in said combined chemotherapy include anastrozole, exemestane, ethinyl estradiol, chlormadinone, goserelin, tamoxifen, bicalutamide, flutamide, buredonizoron, leuprorelin and letrozole.

The biological response modifiers used in said combined chemotherapy include interferon-α, interferon-β, interferon-γ, interleukin 2, ubenimex, dried BCG and Lentinan.

In the combined use of the chemotherapy, a hydrophilic chemotherapeutic agent, or a hydrophobic chemotherapeutic agent may be used by being dissolved or suspended together with carbon dioxide in the liquid in which carbon dioxide is dissolved in accordance with the physical properties of said liquid.

In that case, the dose of the chemotherapeutic agent is reduced than the standard dose, and, while complementing the anti-tumor effect with carbon dioxide, side effects can be reduced. Alternatively, when the dose of the chemotherapeutic agent is the same as the standard one, although usual side effects are observed, the anti-tumor effect can also be more enhanced with carbon dioxide.

The immunotherapies used in combination with the method of treatment of the present invention using a liquid medicine comprising liquid having carbon dioxide dissolved therein, said liquid being administered by using means of liquid injection include cytokine therapy, immunomodulators, and the like as active immunotherapies as well as highly activated NK cell therapy and activated lymphocyte therapy as passive immunotherapies (immune cell therapies).

In the combined use of the immunotherapy, a hydrophilic immunotherapeutic agent, or a hydrophobic immunotherapeutic agent may be used by being dissolved or suspended together with carbon dioxide in the liquid in which carbon dioxide is dissolved in accordance with the physical properties of said liquid. In that case, the dose of the immunotherapeutic agent is reduced than the standard one, and, while complementing the anti-tumor effect with carbon dioxide, side effects can be reduced. Alternatively, when the dose of the immunotherapeutic agent is the same as the standard one, although usual side effects are observed, the anti-tumor effect can also be more enhanced with carbon dioxide.

When the method of treatment of the present invention using a liquid medicine comprising liquid having carbon dioxide dissolved therein, said liquid being administered by using means of liquid injection is used in combination with a non-steroidal anti-inflammatory agent, a steroidal anti-inflammatory agent, an immunosuppressant, or a plasma exchange therapy in the treatment of autoimmune diseases, said liquid medicine may be injected each time the therapy using them is performed, or alternatively said liquid medicine may be injected on the day when the therapy using them is not performed. When said liquid medicine is used in combination with the non-steroidal anti-inflammatory agent, the steroidal anti-inflammatory agent, or the immunosuppressant, they may be concurrently dissolved or suspended in the liquid containing carbon dioxide.

Examples of the non-steroidal anti-inflammatory agents used in combination with the method of treatment of the present invention using a liquid medicine comprising liquid having carbon dioxide dissolved therein, said liquid being administered by using means of liquid injection include salicylic acid-based anti-inflammatory agents such as acetylsalicylic acid, salicylamide, sodium salicylate, diflunisal, and ethenzamide; arylacetic acid-based anti-inflammatory agents such as acemetasin, anfenac sodium, indometacin, etodolac, diclofenac sodium, sulindac, nabumetone, fenbufen, ploglumetacin maleate, and mofezolac; oxicam-based anti-inflammatory agents such as ampiroxicam, tenoxicam, piroxicam, meloxicam, and lornoxicam; propionic acid-based anti-inflammatory agents such as alminoprofen, ibuprofen, oxaprozin, ketoprofen, zaltoprofen, tiaprofenic acid, naproxen, fenoprofen, flurbiprofen, pranoprofen, and loxoprofen sodium; fenamic acid-based anti-inflammatory agents such as tolfenamic acid, flufenamate aluminum, and mefenamic acid; pyrimidine-based anti-inflammatory agents such as bucolome; coxib-based anti-inflammatory agents such as celecoxib, valdecoxib, lumiracoxib, and parecoxib sodium; and basic anti-inflammatory agents such as emorfazone, epirizole, and tiaramide hydrochloride.

Examples of the steroidal anti-inflammatory agents include hydrocortisone succinate, hydrocortisone sodium succinate, prednisolone sodium succinate, methylprednisolone succinate, methylprednisolone sodium succinate, dexamethasone, triamcinolone, triamcinolone acetonide, dexamethasone palmitate, hydrocortisone, prednisolone, betamethasone, methylprednisolone, dexamethasone sodium phosphate, hydrocortisone sodium phosphate, prednisolone sodium phosphate, betamethasone sodium phosphate, cortisone acetate, dexamethasone acetate, paramethasone acetate, halopredone acetate, fludrocortisone acetate, methylpredonisolone acetate, and the like.

Examples of the immunosuppressants include the above steroidal anti-inflammatory agents, alkylating agents such as cyclophosphamide, cytotoxic antibiotics such as anthracycline, dactinomycin, mitomicin c, mithramycin, and bleomycin; cytokine inhibitors such as adalimumab, infliximab, etanercept, and tocilizumab; metabolic antagonists such as azathioprine, mycophenolic acid, mycophenolate mofetil, leflunomide, mercaptopurine, and methotrexate; specific lymphocyte signal transduction inhibitors such as evelolimus, cyclosporine, sirolimus, tacrolimus, and temsirolimus; antibodies against IL-2 receptor protein such as CD3 and CD25; cytokines such as interferon.

In the combined use of a non-steroidal anti-inflammatory agent, a steroidal anti-inflammatory agent, and an immunosuppressant (hereinafter referred to as an agent for autoimmune diseases), a hydrophilic agent for autoimmune diseases, or a hydrophobic agent for autoimmune diseases may be used by being dissolved or suspended together with carbon dioxide in the method of treatment of the present invention using a liquid medicine comprising liquid having carbon dioxide dissolved therein, said liquid being administered by using means of liquid injection in accordance with the physical properties of said liquid. In that case, the dose of the agent for autoimmune diseases is reduced than the standard one, and, while complementing or enhancing the effect with carbon dioxide, side effects can be reduced. Alternatively, when the dose of the agent for autoimmune diseases is the same as the standard one, the effect can also be more enhanced with carbon dioxide.

### EXAMPLES

Next, the present invention is illustrated in more detail by the following Test Examples and Examples. However, the present invention is not limited to these Examples.

### Test Example 1 Antitumor effects (1) on the implanted tumors in rabbits (using a physiological saline solution as a liquid that dissolves carbon dioxide)

### Test Method

Four rabbits (Japanese white domestic rabbits) of 14 weeks old during the third week after implantation of VX2 tumor (control group: three, carbon dioxide group: one) were used for the test. Tumor volume was calculated by the following equation: (Maximum diameter of implanted tumor mass) x (Minimum diameter of implanted tumor mass) x (Minimum diameter of implanted tumor mass) x 1/2.

The measurement of tumor volume was done before the start of the test, and on the sixth day of the test. In the carbon dioxide group, a 5 Fr catheter was inserted from an exposed femoral artery on the side opposite to the tumor up until just above the abdominal aortic bifurcation, connected to the central venous reservoir port, and implanted subcutaneously in the thigh. 25 mL of the physiological saline solution obtained in Example 1 having carbon dioxide dissolved therein was administered at a rate of 5 mL/min from the reservoir port on the first and third days of the test.

The control group was kept in the same manner as in the carbon dioxide group without any medical treatment for the implanted tumor.

### Test Results

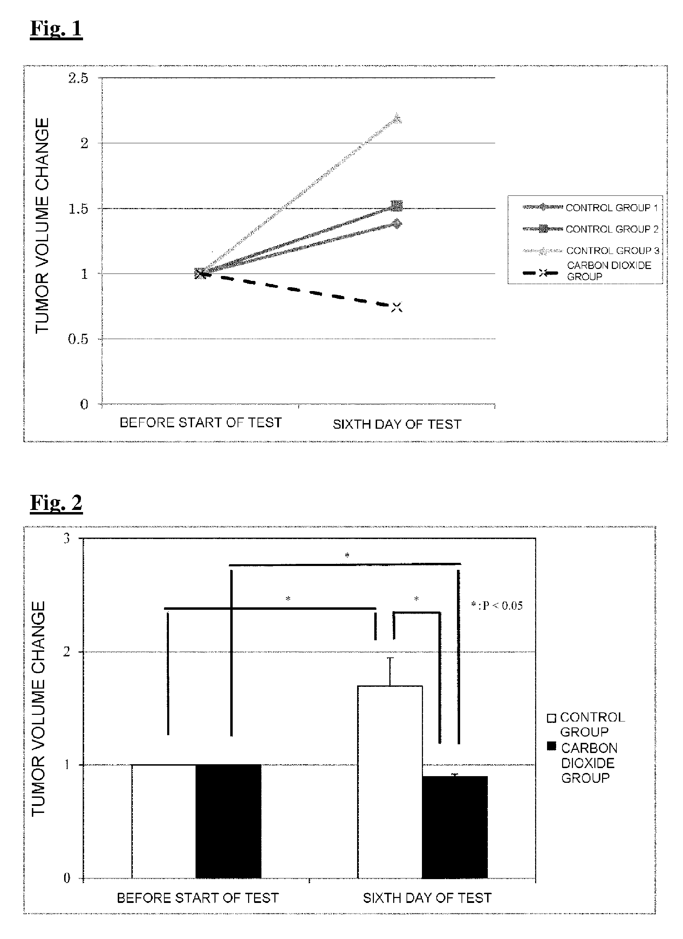

Tumor volumes were respectively increased by 38 %, 52 %, and 119 % in the control group compared to those before the start of the test, while on the other hand, the tumor volume was reduced by 25 % in the carbon dioxide group (FIG. 1). During the test period, there were no deaths in the two groups.

### Test Example 2 Antitumor effects (2) on the implanted tumors in rabbits (using a physiological saline solution as a liquid that dissolves carbon dioxide)

### Test Method

Six rabbits (Japanese white domestic rabbits) of 14 weeks old during the third week after implantation of VX2 tumor (control group: three, carbon dioxide group: three) were used for the test. Tumor volume was calculated by the following equation: (Maximum diameter of implanted tumor mass) x (Minimum diameter of implanted tumor mass) x (Minimum diameter of implanted tumor mass) x 1/2.

The measurement of tumor volume was done before the start of the test, and on the sixth day of the test. In the carbon dioxide group, on the first day of the test, an exposed femoral artery on the same side of the tumor was punctured against the blood flow with a 24G needle, and 50 mL of the physiological saline solution obtained in Example 1 having carbon dioxide dissolved therein was administered at a rate of 5 mL/min by using a syringe.

The control group was kept in the same manner as in the carbon dioxide group without any medical treatment for the implanted tumor.

After completion of the test, the tumor was collected to prepare paraffin sections. Using an apoptosis analysis kit [Apo direct kit™: manufactured by Becton Dickinson Co.], DNA fragments were fluorescent-labeled by the TUNEL method, and DNA quantity was determined using a fluorescent microscope (BZ-8100, manufactured by KEYENCE CO.) to evaluate apoptosis.

### Test Results

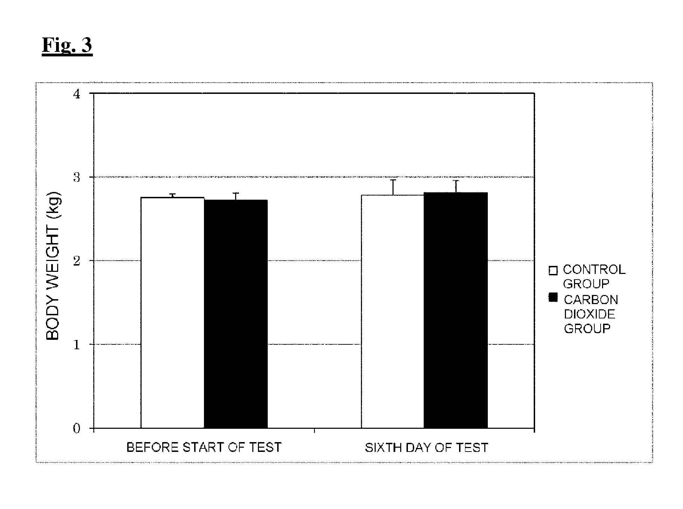

Tumor volumes were increased by about 70 % with a statistically significant difference by t-test in the control group on the sixth day of the test compared to those before the start of the test. On the other hand, tumor volumes were reduced by about 10 % with a statistically significant difference by t-test in the carbon dioxide group, which was about half of the tumor volume of the control group on the sixth day of the test (FIG. 2). During the test period, there were no reductions in body weight in the two groups (FIG. 3). Also, there were no deaths in the two groups.

In the evaluation of apoptosis by the TUNEL method, more DNA fragments were fluorescent-labeled in the tissues of the carbon dioxide group compared to the tissues of the control group, demonstrating that more apoptosis occurred.

### Test Example 3 Antitumor effects (3) on the implanted tumors in rabbits (using a physiological saline solution as a liquid that dissolves carbon dioxide)

### Test Method

Eight rabbits (Japanese white domestic rabbits) of 14 weeks old during the third week after implantation of VX2 tumor (control group: four, carbon dioxide group: four) were used for the test. Tumor volume was calculated by the following equation: (Maximum diameter of implanted tumor mass) x (Minimum diameter of implanted tumor mass) x (Minimum diameter of implanted tumor mass) x 1/2.

The measurement of tumor volume was done before the start of the test, and on the third day of the test. In the carbon dioxide group, on the first day of the test, an exposed femoral artery on the same side of the tumor was punctured against the blood flow with a 24G needle, and 50 mL of the physiological saline solution obtained in Example 1 having carbon dioxide dissolved therein was administered at a rate of 5 mL/min by using a syringe.

The control group was kept in the same manner as in the carbon dioxide group without any medical treatment for the implanted tumor.

After completion of the test, the tumor was collected to prepare paraffin sections. Using an apoptosis analysis kit [Apo direct kit™: manufactured by Becton Dickinson Co.], DNA fragments were fluorescent-labeled by the TUNEL method, and DNA quantity was determined using a fluorescent microscope (BZ-8100, manufactured by KEYENCE CO.) to evaluate apoptosis. Adding to that, proteins have been extracted from the collected tumor tissues and apoptosis was evaluated using Caspase-3 antibody (manufactured by Cell Signaling Technology) by Western blotting method.

### Test Results

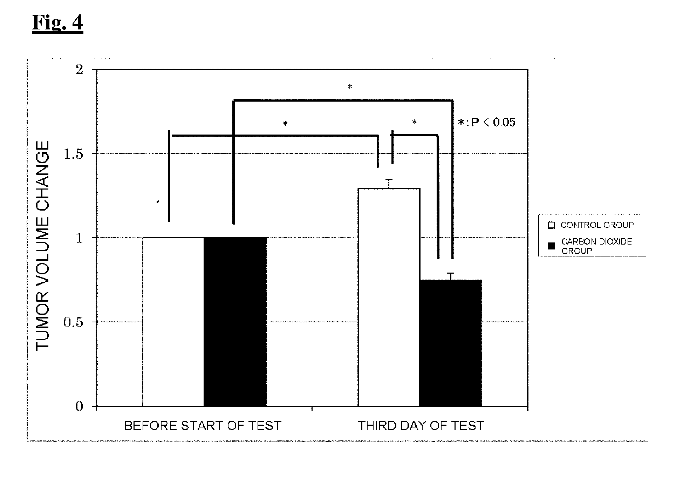

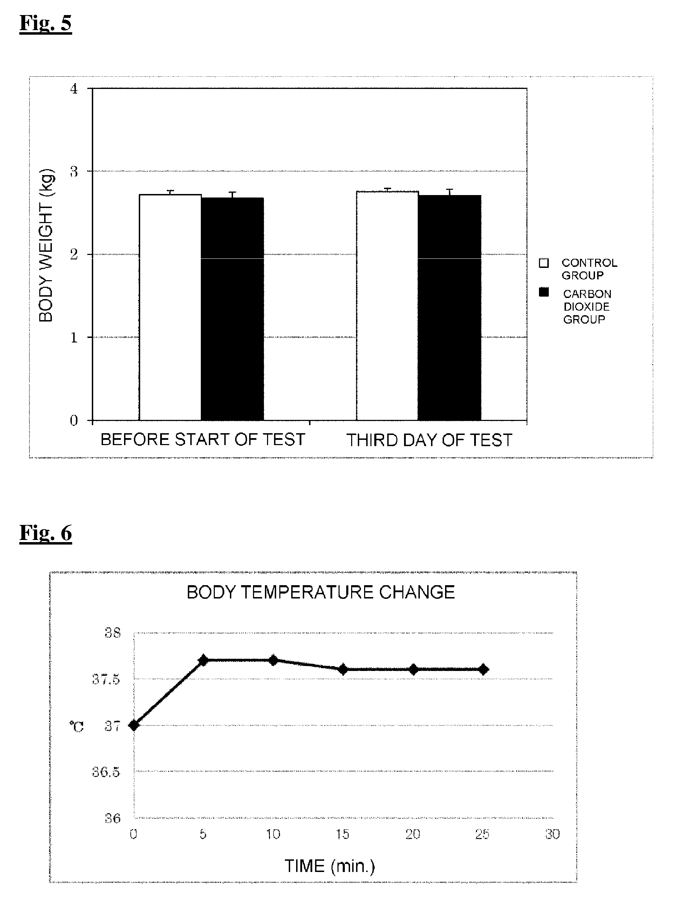

Tumor volumes were increased by about 30 % with a statistically significant difference by t-test in the control group on the third day of the test, compared to those before the start of the test. On the other hand, tumor volumes were reduced by about 25 % with a statistically significant difference by t-test in the carbon dioxide group, which was about 60 % of the tumor volume of the control group on the third day of the test (FIG. 4). During the test period, there were no reductions in body weight in the two groups (FIG. 5). Also, there were no deaths in the two groups.

In the evaluation of apoptosis by the TUNEL method, more DNA fragments were fluorescent-labeled in the tissues of the carbon dioxide group compared to the tissues of the control group, demonstrating that more apoptosis occurred. Furthermore, also from the fact that the amount of inactive form of Caspase-3 (full-length) was reduced and the amount of active form of Caspase-3 (cleaved) was increased, it was demonstrated that more apoptosis occurred in the carbon dioxide group.

### Test Example 4 Safety to healthy pig (using a physiological saline solution as a liquid that dissolves carbon dioxide)

A female experimental mini-pig (Swine W/L) weighing 51 kg was used. A tip of a catheter was placed in the celiac artery, and the physiological saline solution obtained in Example 1 having carbon dioxide dissolved therein was administered at a rate of 100 mL/min. A blood sample was collected every 5 minutes from the right internal carotid artery. The pH, partial pressure of carbon dioxide, and partial pressure of oxygen in blood were measured by a blood gas analyzer (AVL OPTI Critical Care Analyzer), and the body temperature was measured by a thermometer. 2500 mL in total of the physiological saline solution having carbon dioxide dissolved therein was injected.

### Test Results

Although the body temperature was increased by 0.6 °C to 0.7 °C, this was within a range that poses few problems physiologically (FIG. 6).

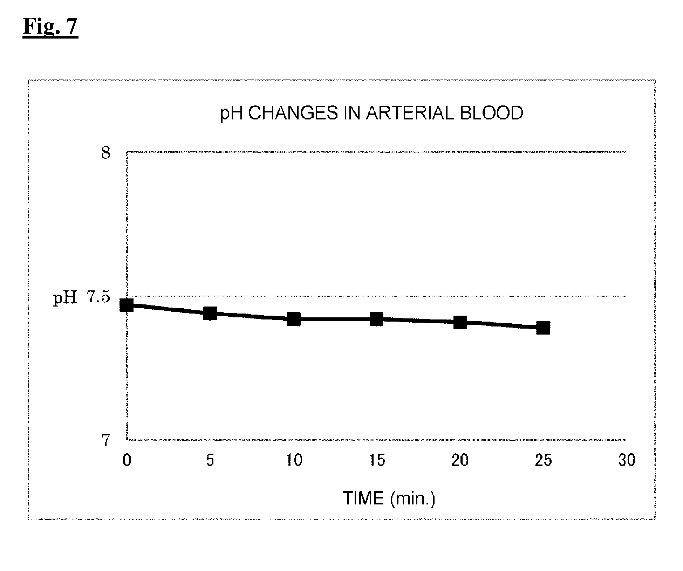

The pH of arterial blood was lowered slightly despite that the physiological saline solution having carbon dioxide dissolved therein had a pH of 4.62, which was within a range that poses few problems physiologically (FIG. 7).

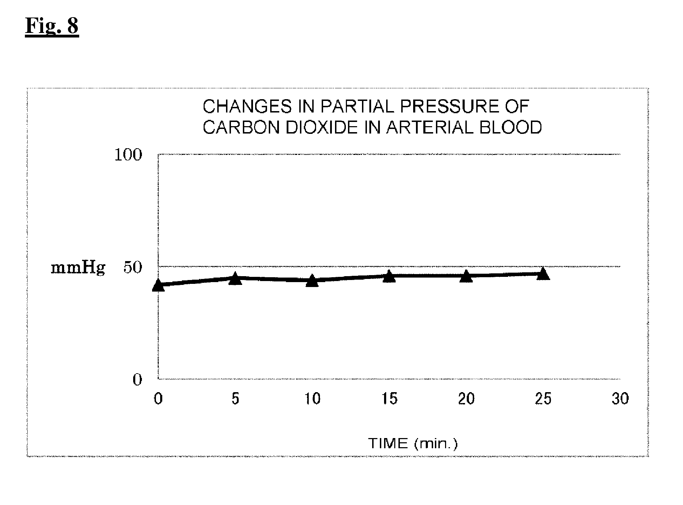

Although the partial pressure of carbon dioxide in arterial blood was increased by about 2 to 6 mmHg, this was within a range that poses few problems physiologically (FIG. 8).

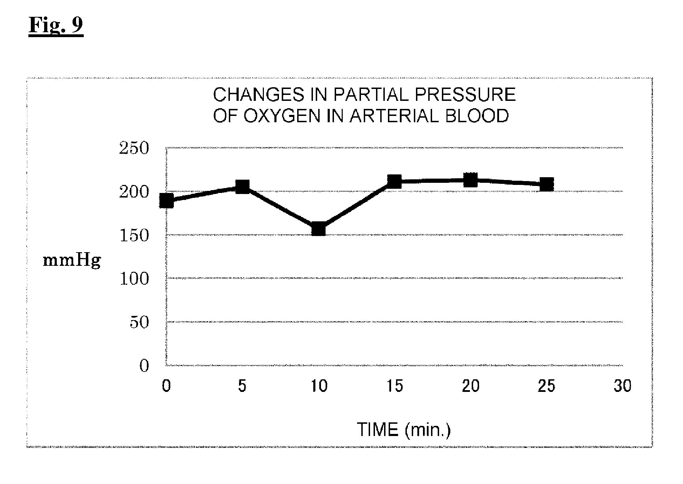

The partial pressure of oxygen in arterial blood was increased by 15 to 25 mmHg except for a value 10 minutes after the start of the test, showing the favorable results that oxygen supply was increased (FIG. 9). It is considered that the value 10 minutes after the start of the test was due to a flawed operation.

### Example 1

A silicone tube was connected to the regulator of the liquefied carbon dioxide cylinder, and an injection needle (diameter: 18G) was attached to the silicone tube. The injection needle was inserted through a rubber plug of a plastic bag containing a physiological saline solution (250 mL; pH 6.34). Another needle for venting excess carbon dioxide was inserted into an upper portion of the plastic bag where there was no physiological saline solution, and carbon dioxide was blown to bubble the solution at a rate of 1 L/min for 5 min to prepare a physiological saline solution having carbon dioxide dissolved therein (pH 4.62).

### Example 2

To 5 mL of venous blood collected from the forearm of a 29 year-old male using a vacuum blood collection tube, 0.5 mL of 3.8 % aqueous solution of sodium citrate for preventing blood coagulation was added to prepare liquid for dissolving carbon dioxide (pH 7.21). A silicone tube was connected to the regulator of the liquefied carbon dioxide cylinder, and an injection needle (diameter: 18G) was attached to the silicone tube. The injection needle was inserted into the blood collection tube, and carbon dioxide was blown to bubble the solution at a rate of 0.5 L/min for 3 min to obtain blood having carbon dioxide dissolved therein (pH 6.83).

### Example 3

A silicone tube was connected to the regulator of the liquefied carbon dioxide cylinder, and an injection needle (diameter: 18G) was attached to the silicone tube. The injection needle was inserted through a rubber plug of a plastic bag containing fat emulsion containing 20 % purified soybean oil (100 mL; product name: Intralipos, manufactured by Otsuka Pharmaceutical Co., Ltd., pH 7.21). Another needle for venting excess carbon dioxide was inserted into an upper portion of the plastic bag where there was no fat emulsion, and carbon dioxide was blown to bubble the emulsion at a rate of 1 L/min for 5 min to prepare a fat emulsion having carbon dioxide dissolved therein (pH 4.67).

As is apparent from the above results, the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection reduce tumors without any side effects such as body weight reductions.

This anti-tumor effect is based on apoptosis induction of tumor cells. A combination of the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection with the existing therapeutic methods for tumors, which have different mechanisms of action and the like, shows synergetic or additive effects, as well as provides an excellent method of treating tumors with few side effects or weak side effects.

Also with respect to autoimmune diseases, which are thought to occur or develop due to reduction or disappearance of the apoptosis induction ability, the liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection are excellent in efficacy and safety.

### INDUSTRIAL APPLICABILITY

The liquid medicine of the present invention comprising liquid having carbon dioxide dissolved therein, said liquid being administered by using means of liquid injection, and the method of treatment of the present invention using a liquid medicine comprising liquid having carbon dioxide dissolved therein, said liquid being administered by using means of liquid injection can reduce or eliminate tumors with few side effects, and can suppress metastasis.

Further, when the liquid medicine of the present invention and the method of treatment of the present invention are used in combination with the surgical therapy, chemotherapy, radiotherapy, and immunotherapy of tumors, the effects can be enhanced or side effects can be reduced compared with monotherapy or multidisciplinary therapy thereof. Similarly, the liquid medicine of the present invention and the method of treatment of the present invention are effective also for autoimmune diseases, and have high safety.

## Claims

1. A liquid medicine comprising a liquid having carbon dioxide dissolved therein, the liquid being adapted to be administered by using means of liquid injection.

2. The liquid medicine according to claim 1,
wherein the means of liquid injection is an active injection in which a pressure is applied so as to be injectable at a speed of 0.1 ml/min or more against the arterial blood pressure when the liquid is injected into an artery.

3. The liquid medicine according to claim 1 or 2,
wherein the means of liquid injection is an active injection using a syringe or a pump.

4. The liquid medicine according to any one of claims 1 to 3,
wherein the liquid that dissolves carbon dioxide is a physiological saline solution.

5. The liquid medicine according to any one of claims 1 to 3,
wherein the liquid that dissolves carbon dioxide is blood compatible with the patient's blood type.

6. The liquid medicine according to any one of claims 1 to 3,
wherein the liquid that dissolves carbon dioxide is a fat emulsion.

7. The liquid medicine according to any one of claims 1 to 3,
whose target disease is a tumor.

8. The liquid medicine according to claim 7,
which is used in combination with one or two or more selected from surgical therapy, chemotherapy, radiotherapy, and immunotherapy of tumors.

9. The liquid medicine according to any one of claims 1 to 3,
whose target disease is an autoimmune disease.

10. The liquid medicine according to claim 9,
which is used in combination with one or two or more of a non-steroidal anti-inflammatory agent, a steroidal anti-inflammatory agent, an immunosuppressant, and a plasma exchange therapy in treatment of autoimmune diseases.

11. A method of treating a tumor,
comprising administering a liquid having carbon dioxide dissolved therein by using means of liquid injection.

12. The method of treatment according to claim 11,
wherein the means of liquid injection is an active injection in which a pressure is applied so as to be injectable at a speed of 0.1 ml/min or more against the arterial blood pressure when the liquid is injected into an artery.

13. The method of treatment according to claim 12,
wherein the means of liquid injection is an active injection using a syringe or a pump.

14. The method of treatment according to claim 11,
wherein the liquid that dissolves carbon dioxide is a physiological saline solution.

15. The method of treatment according to claim 11,
wherein the liquid that dissolves carbon dioxide is blood compatible with the patient's blood type.

16. The method of treatment according to claim 11,
wherein the liquid that dissolves carbon dioxide is a fat emulsion.

17. The method of treatment according to claim 11,
which is used in combination with one or two or more selected from surgical therapy, chemotherapy, radiotherapy, and immunotherapy of tumors.

18. A method of treating an autoimmune disease,
comprising administering a liquid having carbon dioxide dissolved therein by using means of liquid injection.

19. The method of treatment according to claim 18,
wherein the means of liquid injection is an active injection in which a pressure is applied so as to be injectable at a speed of 0.1 ml/min or more against the arterial blood pressure when the liquid is injected into an artery.

20. The method of treatment according to claim 19,
wherein the means of liquid injection is an active injection using a syringe or a pump.

21. The method of treatment according to claim 18,
wherein the liquid that dissolves carbon dioxide is a physiological saline solution.

22. The method of treatment according to claim 18,
wherein the liquid that dissolves carbon dioxide is blood compatible with the patient's blood type.

23. The method of treatment according to claim 18,
wherein the liquid that dissolves carbon dioxide is fat emulsion.

24. The method of treatment according to claim 19,
which is used in combination with one or two or more of a non-steroidal anti-inflammatory agent, a steroidal anti-inflammatory agent, an immunosuppressant, and a plasma exchange therapy in treatment of autoimmune diseases.
